# EUROPEAN PATENT APPLICATION

(11) **EP 4 201 367 A1**
(43) Date of publication of application: **28.06.2023**
(21) Application number: 21857245.1
(22) Date of filing: 13.05.2021
(51) Int. Cl.: A61C 7/00

(54) **ORTHODONTICS ASSISTING DEVICE**

(30) Priority: 19.08.2020 CN 202010838339
(71) Applicant: SUZHOU BESHONE INTELLIGENT TECHNOLOGY CO., LTD., Suzhou, Jiangsu 215123 (CN)
(72) Inventor: ZHAO, Peng, Suzhou, Jiangsu 215123 (CN); ZHAO, Lei, Suzhou, Jiangsu 215123 (CN); DUAN, Xiaohua, Suzhou, Jiangsu 215123 (CN); HUANG, Fengrong, Suzhou, Jiangsu 215123 (CN); HU, Yuehua, Suzhou, Jiangsu 215123 (CN); MA, Wenhui, Suzhou, Jiangsu 215123 (CN); GUAN, Jianjun, Suzhou, Jiangsu 215123 (CN); XIN, Jinxing, Suzhou, Jiangsu 215123 (CN); LIU, Juan, Suzhou, Jiangsu 215123 (CN)
(74) Representative: Bryn Aarflot AS
(86) International application number: PCT/CN2021/093705
(87) International publication number: WO 2022/037144

(57) **Abstract**

An orthodontics assisting device, comprising an occlusion mechanism (100), a control mechanism, a vibration driving mechanism (510) and an optical driving mechanism (450); the occlusion mechanism (100) can be placed in the oral cavity, and the occlusion mechanism (100) is provided with a light generator (400); the occlusion mechanism (100) is connected to the vibration driving mechanism (510), and the light generator (400) is connected to the optical driving mechanism (450); the vibration driving mechanism (510) and the optical driving mechanism (450) are electrically connected to the control mechanism; and the control mechanism is used for controlling the vibration driving mechanism (510) to drive the occlusion mechanism (100) to generate vibration, and controlling the optical driving mechanism (450) to drive the light generator (400) to generate light. The light generator (400) can generate light of a specific band under the control of the optical driving mechanism (450). The orthodontics assisting device increases the movement speed of teeth and reduces the pain of teeth by means of vibration and illumination, so as to shorten the period of orthodontics and improve the comfort.

## Description

### Cross-References to Related Application

The present disclosure claims the priority to the Chinese patent application with the filling number 2020108383394 and entitled "Orthodontics Assisting Device" filed with the Chinese Patent Office on August 19, 2020, the entire contents of which are incorporated herein by reference.

### Technical Field

The present disclosure relates to the field of orthodontics, in particular to an orthodontics assisting device.

### Background Art

Orthodontics is the straightening of teeth and the removal of malocclusions and malformations. Orthodontics may achieve the effect of teeth beautification.

The duration of orthodontic treatment varies according to the degree of tooth malformation, but even for children with relatively mild malformations, it takes at least 6 months, while adult orthodontics usually takes 2 to 3 years. The fundamental reason is that the current orthodontic method uses the "mechanical" means of aligner, and the "mechanical" structure of the aligner is adjusted at intervals to finally achieve a neat and beautiful arrangement of teeth. At the same time, after the mechanical adjustment for the aligner, the teeth will suffer obvious pain.

To sum up, orthodontics currently takes a long time and the process is painful.

### Summary

The object of the present disclosure includes, for example, providing an orthodontics assisting device capable of illuminating light on the periodontium and vibrating the teeth, thereby shortening the period of orthodontics and reducing the tooth pain.

The present disclosure is implemented as follows.

An orthodontics assisting device, which may include an occlusion mechanism, a control mechanism, a vibration driving mechanism, and an optical driving mechanism, wherein the occlusion mechanism is configured to be capable of being placed in the oral cavity, the occlusion mechanism may be equipped with a light generator, and the light generator may correspond to the position of the periodontium in the oral cavity; the occlusion mechanism may be connected with the vibration driving mechanism, and the light generator of the occlusion mechanism may be connected with the optical driving mechanism; the vibration driving mechanism and the optical driving mechanism may be electrically connected to the control mechanism, respectively; the control mechanism may be configured to control the vibration driving mechanism to drive the occlusion mechanism to vibrate; and the control mechanism may be configured to control the optical driving mechanism to drive the light generator to generate light.

Optionally, the control mechanism may be configured to control the vibration driving mechanism to drive the occlusion mechanism to produce vibrations changed in amplitude, frequency and time; the control mechanism may be configured to control the optical driving mechanism to drive the light generator to generate light changed in amplitude, frequency and time; and the control mechanism may generate vibration signals and optical signals that cooperate with each other, to control the operation of the vibration driving mechanism and the optical driving mechanism, respectively.

Optionally, the vibration driving signal generated by the control mechanism may generate vibration-interval reminder information at a certain time interval.

Optionally, the light generator may emit light with a set wavelength to increase the secretion of adenosine triphosphate (ATP) enzyme in periodontium cells, thereby promoting the generation of ATP and accelerating the growth of periodontium cells. The light generator may emit light with a set wavelength to promote the growth of newly born capillaries around the periodontium and provide more nutrients for the growth of periodontium cells. Vibration stimulation may promote the growth of periodontal membrane. Light with a set wavelength and vibration stimulation may inhibit the secretion of inflammatory cytokines including cyclooxygenase-2 and interleukin-1, thereby having an anti-inflammatory and analgesic effect.

Preferably, the light emitted by the light generator has a wavelength of 650 nm-950 nm.

In one or more feasible embodiments, the orthodontics assisting device may further include a main body housing, both the control mechanism and the vibration driving mechanism may be installed in the main body housing, and the vibration driving mechanism and the occlusion mechanism may be connected through a vibration conducting sheet.

In one or more feasible embodiments, the main body housing is made of ABS+PC synthetic material.

In one or more feasible embodiments, the main body housing may include a first half casing and a second half casing, and the edge region of the first half casing is snap-fitted to the edge region of the second half casing.

Optionally, a snapping slot may be provided on the side of the first half casing, and a fixture block may be provided on the side of the second half casing, and the fixture block may extend into the snapping slot.

In one or more feasible embodiments, the occlusion mechanism may be provided with an accommodating cavity, and part of the structure of the vibration conducting sheet is embedded in the accommodating cavity. In this case, the contact area between the vibration conducting sheet and the occlusion mechanism is larger, and thus the vibration is transmitted more evenly.

In one or more feasible embodiments, the orthodontics assisting device may further include a power supply mechanism, and the power supply mechanism is respectively connected with the control mechanism, the vibration driving mechanism and the optical driving mechanism. The power supply mechanism may include a battery pack, a voltage stabilizing circuit and a power adjustment circuit, wherein the battery pack is electrically connected to the voltage stabilizing circuit and the power adjustment circuit, respectively, and the power supply mechanism may be configured to provide electric energy for the control mechanism, the vibration driving mechanism and the light generator, and provide protection against undervoltage, overcurrent, overvoltage and so on.

In one or more feasible embodiments, the occlusion mechanism may include a base plate and an occlusion plate, wherein the first side surface of the base plate may be configured to match the periodontal shape in the oral cavity, and the corners of the base plate and the occlusion plate may all be provided with chamfers, wherein the chamfers are rounded chamfers or gradual chamfers composed of multiple planes; and the occlusion plate is connected to the base plate at a set angle, and both the light generator and the occlusion plate may be installed on the first side surface of the base plate. The base plate and the occlusion plate have a certain degree of softness, which may adapt to oral cavity of different shapes and sizes.

Optionally, two opposite edges of the base plate may be respectively provided with a V-shaped notch recessed from the outside to the inside, and the two V-shaped notches may be respectively located on two sides of the occlusion plate.

Optionally, the two V-shaped notches are arranged symmetrically with respect to the occlusion plate. A bending part of the V-shaped notch may be transitioned in a manner of arc.

In one or more feasible embodiments, the occlusion plate may divide the base plate into an upper region (upper-layer region) and a lower region (lower-layer region), the upper region may be configured to cover the entire upper periodontium, and the lower region may be configured to cover the entire lower periodontium; and the light generator may include a plurality of light-emitting elements, and both the upper region and the lower region may be provided with several light-emitting elements.

In one or more feasible embodiments, the occlusion plate may also be connected with an auxiliary lighting board, and the auxiliary lighting board may also be provided with the light-emitting elements, and the light-emitting element of the auxiliary lighting board is configured to illuminate the periodontal region toward the inner side of the oral cavity. In this way, the oral tissue may be illuminated from multiple angles.

A plurality of light-emitting elements may form several light-emitting matrices, and the plurality of light-emitting elements may be respectively connected with the optical driving mechanism; and the optical driving mechanism may independently control the amplitude, frequency and time of light emitted from each light-emitting element or each light-emitting matrix.

It is set so that during using, the optical driving mechanism may individually control one or several of light-emitting elements or light-emitting matrices, or the optical driving mechanism may synchronously control several light-emitting elements in one or several light-emitting matrices. In this way, different tissue sites in the oral cavity may be irradiated separately.

In one or more feasible embodiments, the occlusion mechanism may be provided with a first insertion part, and the main body housing may be provided with a second insertion part, and the second insertion part may be inserted into a hole of the first insertion part. The second insertion part may have an inner cavity, and the vibration driving mechanism may be installed in the inner cavity. In this case, the distance between the vibration driving mechanism and the occlusion mechanism is closer, so that the length of the vibration conducting sheet may be set shorter, thereby reducing the energy loss in the process of vibration transmission.

In one or more feasible embodiments, the orthodontics assisting device may further include a wireless transmitting module, the wireless transmitting module is electrically connected to the control mechanism, wherein the wireless transmitting module may be configured to be in signal connection with the mobile terminal. The mobile terminal is at least configured to input control instructions to control the control mechanism, and to record and display relevant state information such as amplitude, frequency and period of the vibration driving mechanism and the optical driving mechanism.

Preferably, the wireless transmitting module includes a Bluetooth module, a Wi-Fi module, a cellular mobile communication module or other wireless transmitting modules.

Optionally, when the control mechanism includes a circuit board, a Bluetooth module, a Wi-Fi module, a cellular mobile communication module or other wireless transmitting modules are integrated on the circuit board.

In one or more feasible embodiments, the control mechanism may be provided with a control switch, and the control switch may be configured to switch working modes by the control mechanism, wherein the working modes at least include vibration mode, lighting mode and combined vibration-and-lighting mode.

Optionally, the control mechanism may also be connected with an indicator light.

The indicator light is for status indication, and the control mechanism may send control signals to the indicator light, such that the indicator light, through changes on the light color and the flickering, indicates the vibration state, lighting state, combined lighting-and-vibration state, standby state, undervoltage state, time-interval prompt information and other status.

The indicator light may be installed on the surface of the first casing and the surface of the second casing.

Alternatively, the indicator light may be installed in the control mechanism, that is, the indicator light is located inside the main body housing, and the light from the indicator light may pass through the main body housing. Specifically, the main body housing may be made of transparent material, and the inner side of the main body housing is coated with a light-shielding layer, while the region opposite to the indicator light is not coated with a light-shielding layer, so that only the indicator light may be seen outside the main body housing without other structures. Alternatively, a through hole is provided on the main body housing, and the light from the indicator light may emit through the through hole. Alternatively, a pressing portion is provided on the region of main body housing relative to the control switch. Specifically, an arc-shaped through hole is provided on the main body housing, and the region formed by the arc-shaped through hole is the pressing portion. Part of the structure of the pressing portion is connected with other parts of the main body housing and part of the structure acts as a free end, which makes it possible to press the pressing portion inward, thereby the pressing portion presses the control switch located inside the main body housing. The indicator light may be installed around the control switch, so that the light from the indicator light emits through the arc-shaped through hole.

Optionally, the vibration mode may also include multiple vibration levels, and different vibration levels correspond to different vibration frequencies or different vibration levels correspond to different amplitudes. The lighting mode may also include multiple lighting levels, and different lighting levels correspond to different illuminated regions, or different lighting levels correspond to light of different wavelengths.

The present disclosure at least includes the following beneficial effects.

During use, the occlusion mechanism is placed in the oral cavity, and the occlusion mechanism is bitten by the teeth. In use, the controller controls the light generator to emit light through the optical driving mechanism, and the light illuminates the oral tissue, which stimulates the CCO (cytochrome C oxidase) of the oral tissue (especially the periodontium directly facing the light generator) to enhance the absorption of photons and proton pumping, and to increase the generation of ATP, wherein the increase of cellular energy leads to the higher metabolic activity of cells. At the same time, lighting (illumination) may promote the generation of nitric oxide, accelerate the growth of newly born capillaries, provide more nutrient supply for periodontium, improve the metabolic activity of cells, and accelerate the osteogenesis and osteoclasis of alveolar bone, so that the teeth are able to move faster under the external force. The controller may control the vibration driving mechanism to drive the movement of the occlusion mechanism, thereby driving the teeth to vibrate, improving the growth and metabolism of the periodontal membrane, and increasing the moving speed of the teeth.

The illumination of light on the gingival tissue and high-frequency vibration may inhibit the secretion of inflammatory cytokines such as cyclooxygenase-2 and interleukin-1, so as to achieve anti-inflammatory and analgesic effect.

To sum up, when using the orthodontics assisting device provided by the present disclosure in the process of orthodontics, a single vibration mode, a lighting mode, or a combined vibration-and-lighting mode may be selected according to the needs, so as to increase the speed of the tooth movement, thereby speeding up the orthodontic speed and reducing the tooth pain.

### Brief Description of Drawings

In order to illustrate the technical solutions of the examples of the present disclosure more clearly, the following will briefly introduce the drawings used in the examples. It should be understood that the following drawings only show some examples of the present disclosure, so they should not be regarded as a limitation on the scope, and those of ordinary skilled in the art may also obtain other related drawings based on these drawings without inventive effort.
Fig. 1 is a structural schematic view I of an orthodontics assisting device provided by the embodiment of the present disclosure;
Fig. 2 is a structural schematic view II of the orthodontics assisting device provided by the embodiment of the present disclosure;
Fig. 3 is a structural schematic view III of the orthodontics assisting device provided by the embodiment of the present disclosure;
Fig. 4 is a structural schematic view VI of the orthodontics assisting device provided by the embodiment of the present disclosure;
Fig. 5 is a structural schematic view V of the orthodontics assisting device provided by the embodiment of the present disclosure;
Fig. 6 is a schematic view I of the relative positional relationship between a vibration conducting sheet and a light generator provided by the embodiment of the present disclosure;
Fig. 7 is a schematic view II of the relative positional relationship between the vibration conducting sheet and the light generator provided by the embodiment of the present disclosure;
Fig. 8 is a schematic view of the relative positional relationship between a second half casing, a control mechanism, a power supply mechanism and a occlusion mechanism provided by the embodiment of the present disclosure;
Fig. 9 is a schematic view of the relative positional relationship between the control mechanism, the power supply mechanism and the occlusion mechanism provided by the embodiment of the present disclosure;
Fig. 10 is a structural schematic view of a control mechanism provided in an embodiment of the present disclosure;
Fig. 11 is a structural schematic view I of a first half casing provided by the embodiment of the present disclosure;
Fig. 12 is a structural schematic view II of the first half casing provided by the embodiment of the present disclosure;
Fig. 13 is a schematic view I of the relative positional relationship between a second half casing and the occlusion mechanism provided by the example of the present disclosure;
Fig. 14 is a schematic view II of the relative positional relationship between the second half casing and the occlusion mechanism provided by the embodiment of the present disclosure;
Fig. 15 is a schematic view of grouping control of the light generator provided by another embodiment of the present disclosure;
Fig. 16 is a structural schematic view I of an orthodontics assisting device provided by another embodiment of the present disclosure;
Fig. 17 is a structural schematic view II of an orthodontics assisting device provided by another embodiment of the present disclosure;
Fig. 18 is a structural schematic view III of an orthodontics assisting device provided by another embodiment of the present disclosure;
Fig. 19 is a structural schematic view VI of an orthodontics assisting device provided by another embodiment of the present disclosure; and
Fig. 20 is a structural schematic view V of an orthodontics assisting device provided by another embodiment of the present disclosure.

Reference signs: 100-occlusion mechanism; 110-base plate; 111-first side surface; 112-second side surface; 113-V-shaped notch; 114-chamfer; 120-occlusion plate; 121-limit protrusion; 130-first insertion portion; 131-first insertion end; 132-second insertion end; 140-auxiliary lighting board; 200-main body housing; 210-first half casing; 211-pressing portion; 212-protruding block; 213-snapping slot; 214-limiting ring; 215-limiting plug; 216-limiting post; 220-second half casing; 221-first positioning post; 222-second positioning post; 223-through hole; 224-fixture block; 225-limiting slot; 230-second insertion portion; 310-PCB; 311-first positioning hole; 312-limiting opening; 320-wireless transmitting module; 330-board-to-board connector; 340-control switch; 350-indicator light; 360-probe; 400-light generator; 410-first light-emitting element; 420-second light-emitting element; 430-flexible circuit board; 440-third light-emitting element; 450-optical driving mechanism; 510-vibration driving mechanism; 511-third positioning hole; 520-vibration conducting sheet; 521-first segment; 522-second segment; 523-second positioning hole; 600-power supply mechanism.

### Detailed Description of Embodiments

In order to make the purposes, technical solutions and advantages of the embodiments of the present disclosure clearer, the technical solutions in the embodiments of the present disclosure will be clearly and completely described below in conjunction with the drawings in the embodiments of the present disclosure. Obviously, the described embodiments are a part of the embodiments of the present disclosure, not all of them. The components of the embodiments of the present disclosure generally described and illustrated in the figures herein may be arranged and designed in a variety of different configurations. Accordingly, the following detailed description of the embodiments of the present disclosure provided in the drawings is not intended to limit the scope of the claimed disclosure, but merely represents selected embodiments of the present disclosure. Based on the embodiments in the present disclosure, all other embodiments obtained by persons of ordinary skill in the art without inventive efforts fall within the protection scope of the present disclosure.

It should be noted that like reference signs and letters denote similar items in the following drawings, therefore, once an item is defined in one figure, it does not require further definition and explanation in subsequent figures.

In the description of the present disclosure, it should be noted that the terms "first", "second", "third" and so on are only used for distinguishing descriptions, and should not be understood as indicating or implying relative importance. Furthermore, the terms "horizontal", "vertical" and the like do not imply that a component is required to be absolutely horizontal or vertical, but may be slightly inclined. For example, "horizontal" only means that its direction is more horizontal than "vertical", and it does not mean that the structure must be completely horizontal, but may be slightly inclined.

In the description of the present disclosure, it should also be noted that, unless otherwise clearly stipulated and limited, the terms "provide", "install", "connect", and "attach" should be understood in a broad sense, for example, it may be a fixed connection, may also be a detachable connection or an integral connection; it may be a mechanical connection or an electrical connection; it may be a direct connection or an indirect connection through an intermediary, or the internal communication between two components. Those of ordinary skill in the art may understand the specific meanings of the above terms in the present disclosure in specific situations.

Some embodiments of the present disclosure will be described in detail below in conjunction with the drawings. In the absence of conflict, the following embodiments and features in the embodiments may be combined with each other.

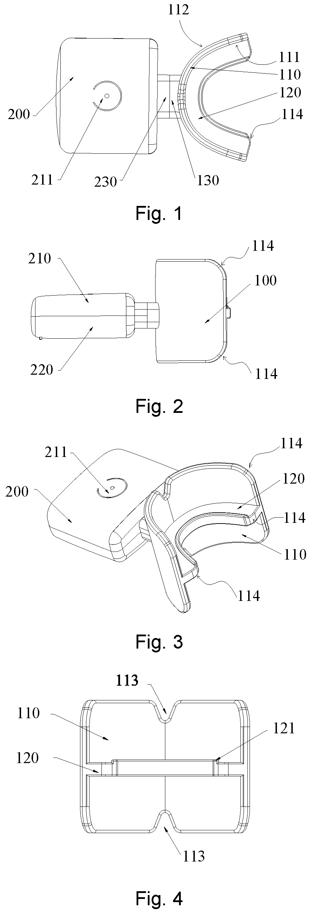

Referring to Fig. 1-Fig. 7, an embodiment of the present disclosure provides an orthodontics assisting device, which includes an occlusion mechanism 100, a control mechanism, a vibration driving mechanism 510 and an optical driving mechanism 450, and the occlusion mechanism 100 is configured to be able to be placed in the oral cavity. The occlusion mechanism 100 is equipped with a light generator 400, wherein the light generator 400 is connected to the optical driving mechanism 450. The light generator 400 corresponds to the position of the periodontium in the oral cavity. The occlusion mechanism 100 is connected to the vibration driving mechanism 510, wherein the vibration driving mechanism 510 and the light generator 400 are respectively electrically connected to the control mechanism, and the control mechanism is configured to control the vibration driving mechanism 510 to drive the occlusion mechanism 100 to vibrate 510. The control mechanism is also configured to control the optical driving mechanism 450 to drive the light generator 400 to emit light.

Optionally, the control mechanism may control any one or more of vibration amplitude, frequency and time generated by the vibration driving mechanism 510 to change, and may change the amplitude, frequency and time of the light emitted by the light generator 400. The control mechanism may generate vibration signals and optical signals that are cooperated with each other to control the operation of the vibration driving mechanism 510 and the optical driving mechanism 450, respectively.

Specifically, the orthodontics assisting device provided in the present disclosure includes at least three working modes, namely: vibration mode, lighting mode, and combined vibration-and-lighting mode.

When the orthodontics assisting device is in the vibration mode, the vibration driving mechanism 510 is activated and drives the occlusion mechanism 100 to vibrate. At this time, the light generator 400 is turned off.

When the orthodontics assisting device is in the lighting mode, the light generator 400 emits the set light. At this time, the vibration driving mechanism 510 is turned off.

When the orthodontics assisting device is in the combined vibration-and-lighting mode, the vibration driving mechanism 510 is activated, and at the same time, the light generator 400 emits the set light.

The light generator 400 emits light to illuminate the oral cavity for adjuvant therapy based on the principle of using photobiomodulation. The light generator 400 emits light with a set wavelength to illuminate the tissues in the oral cavity, thereby stimulating the CCO (cytochrome C oxidase) of the tissues in the oral cavity (especially the periodontium directly facing the light generator 400) to enhance the absorption of photons, and proton pumping, and increase the generation of ATP, in which the increase of cellular energy leads to the higher metabolic activity of cells. At the same time, light may promote the generation of nitric oxide, accelerate the growth of newly born capillaries, provide more nutrient supply for periodontium, improve the metabolic activity of cells and accelerate the osteogenesis and osteoclasis of alveolar bone, so that the teeth are able to move faster under the external force.

In the combined vibration-and-lighting mode, the vibration driving mechanism 510 generates a mechanical signal with a fixed frequency and acceleration, to act on the tooth, which is combined with the light emitted by the light generator 400, so as to act on the tooth together to improve the tooth movement speed.

In a preferred example of the present embodiment, the light emitted by the light generator 400 has a wavelength of 650 nm-950 nm.

In a preferred example of the present embodiment, the vibration driving mechanism 510 includes a motor, preferably a high-frequency micro vibration motor.

In a feasible embodiment, in order to facilitate the installation of the control mechanism and the vibration driving mechanism 510, the orthodontics assisting device further includes a main body housing 200, both the control mechanism and the vibration driving mechanism 510 are installed inside the main body housing 200, and the vibration driving mechanism 510 is connected with the occlusion mechanism 100 through the vibration conducting sheet 520. In this way, the configuration of main body housing 200 provides a larger installation space for the control mechanism and the vibration driving mechanism 510, and the main body housing 200 also provides better protection for the control mechanism and the vibration driving mechanism 510.

The main body housing 200 is made of hard material, so as to provide enhanced protection for structures (e.g., control mechanism and vibration driving mechanism 510) mounted on the inside thereof.

Preferably, the main body housing 200 is made of ABS+PC synthetic material, so that the main body housing 200 is lighter, easier to carry and labor-saving when hold by hand, while protecting the control mechanism and the vibration driving mechanism 510 mounted on the inside thereof.

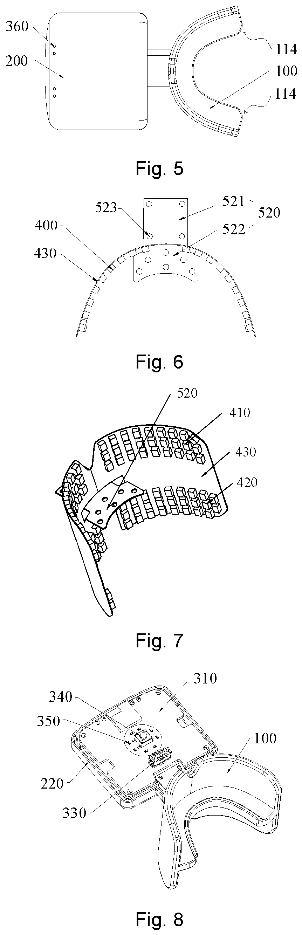

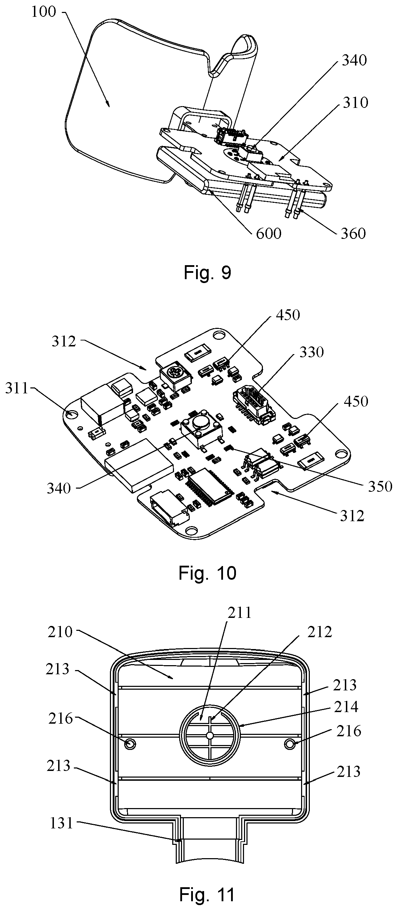

As shown in Fig. 8 and Fig. 9, in a feasible embodiment, the orthodontics assisting device further includes a power supply mechanism 600, and the power supply mechanism 600 is respectively connected with the control mechanism, the vibration driving mechanism 510 and the optical driving mechanism 450. The power supply mechanism 600 is configured to supply power to the control mechanism, the vibration driving mechanism 510 and the optical driving mechanism 450. The power supply mechanism 600 may be a storage battery.

The power supply mechanism 600 is installed in the main body housing 200. In a specific embodiment, the power supply mechanism 600 is detachably installed in the main body housing 200. In this manner, when the power of the power supply mechanism 600 is exhausted, the power supply mechanism 600 will be taken out and replaced with a new one (or one with more power) for continuous using.

In another specific embodiment, the power supply mechanism 600 is a charging power supply. The charging method of the power supply mechanism 600 may through a charging cable, or the charging method of the power supply mechanism 600 may be wireless charging. For example, the power supply mechanism 600 has charging contacts, and the main body housing 200 is provided with a through hole 223 to expose the charging contacts. The charging contacts are used to contact the charging terminals (such as charging electrodes) on the charging stand, so as to charge the charging power source through the charging stand. Specifically, the charging contacts may be provided inside the main body housing 200, and the charging terminals on the charging stand extend into the main body casing 200 to contact the charging contacts; or the end portion of the charging contact is flush with the outer surface of the main body housing 200; or the end portion of the charging contact protrudes from the main body housing 200 and the length of the part of the structure protruding from the main body housing 200 is preferably less than 1 mm.

As shown in Fig. 1-Fig. 4, in a feasible embodiment, the occlusion mechanism 100 includes a base plate 110 and an occlusion plate 120, wherein a first side surface 111 of the base plate 110 is configured to match the periodontal shape in the oral cavity; and the corners of both the base plate 110 and the occlusion plate 120 are provided with chamfers 114, wherein the occlusion plate 120 is connected to the base plate 110 at a set angle. The light generator 400 and the occlusion plate 120 are installed on the first side surface 111 of the base plate 110. Specifically, the chamfer 114 may be a rounded chamfer or a gradual chamfer composed of multiple planes.

During use, the occlusion mechanism 100 is placed inside the oral cavity such that the occlusion plate 120 is positioned between the upper and lower teeth, and the base plate 110 is positioned outside the teeth.

As shown in Fig. 1, the chamfer 114 is a rounded chamfer, which is formed at the corners of edge on the side of the occlusion plate 120 away from the base plate 110; and as shown in Fig. 2, the chamfer 114 is a rounded chamfer which formed at the edge corner of the base plate 110. The rounded chamfer means that the edge of the chamfer 114 is a smooth arc surface, so as to improve the comfort level when the occlusion mechanism 100 is put into the oral cavity; and after the occlusion mechanism 100 is put into the oral cavity, the chamfer 114 makes the structure of the base plate 110 and the structure and the shape of the occlusion plate 120 more compatible with the inner space of the oral cavity, which allows for the support to the upper and lower lips and improves the fitting degree between the base plate 110 to the teeth and periodontium (e.g. gingiva), thus bringing the light generator 400 closer to the teeth and periodontium (e.g. gingiva) and improving the lighting effect.

As shown in Fig. 4, the plate surface of the base plate 110 is an arc surface, and a V-shaped notch 113 is arranged on the base plate 110, wherein the V-shaped notch 113 is located at the edge of the base plate 110. The setting of the V-shaped notch 113 facilitates the further bending of the base plate 110 in the oral cavity, so as to better adapt to the shape of the oral cavity. Specifically, a bending part of the V-shaped notch 113 is smoothly transitioned.

For example, in Fig. 4, V-shaped notches 113 are respectively provided in the middle region of the upper edge of the base plate 110 and the middle region of the lower edge of the base plate 110, and the two V-shaped notches 113 are symmetrically arranged with respect to the occlusion plate 120.

Preferably, the occlusion plate 120 is provided with a limit protrusion 121, wherein the limit protrusion 121 is located on a side of the occlusion plate 120 away from the base plate 110. As shown in Fig. 3 and Fig. 4, the limit protrusion 121 is disposed on the edge of the top surface of the occlusion plate 120.

Certainly, in another arrangement manner, the limit protrusion 121 is disposed on the bottom edge of the occlusion plate 120; or the limit protrusions 121 are disposed on both the top edge and the bottom edge of the occlusion plate 120.

In a preferred embodiment, the occlusion plate 120 divides the base plate 110 into an upper region and a lower region, wherein the upper region is configured to be opposite to the upper periodontium, and the lower region is configured to be opposite to the lower periodontium; and the light generator 400 includes a plurality of light-emitting elements, and the upper region and the lower region are both provided with several light-emitting elements.

Optionally, the upper region is configured to be able to cover the entire upper periodontium, and the lower region is configured to be able to cover the entire lower periodontium.

A plurality of light-emitting elements may form one or more light-emitting matrices, and one light-emitting matrix includes one or more than one light-emitting elements.

Each light-emitting element is respectively connected with the optical driving mechanism 450, the optical driving mechanism 450 may independently control each light-emitting element. The optical driving mechanism 450 may also control each light-emitting matrix to synchronously control light-emitting elements in the light-emitting matrix.

For example, as shown in Fig. 7, all light-emitting elements on the base plate 110 may be divided into two light-emitting matrices. Specifically, all light-emitting elements located in the upper region belong to the same light-emitting matrix, and all light-emitting elements located in the lower region belong to the same light-emitting matrix. For the convenience of distinction, the light-emitting element located in the upper region is referred to as the first light-emitting element 410, and the light-emitting element located in the lower region is referred to as the second light-emitting element 420.

In this case, when using the lighting mode, it is possible to only make the first light-emitting element 410 emit light to illuminate only the upper periodontal-tissue region; or only make the second light-emitting element 420 emit light to illuminate only the lower periodontal-tissue region; or both the first light-emitting element 410 and the second light-emitting element 420 can emit light to simultaneously illuminate the upper periodontal-tissue region and the lower periodontal-tissue region.

Optionally, in a feasible embodiment, the multiple first light-emitting elements 410 located in the upper region are divided into several first light-emitting matrices, and the multiple second light-emitting elements 420 located in the lower region are divided into several second light-emitting matrices. The control mechanism may control each first light-emitting matrix and each second light-emitting matrix. The control states of the multiple first light-emitting elements 410 or second light-emitting elements 420 in each light-emitting matrix are consistent. In this way, it is possible to illuminate only partial region of the upper periodontium, or illuminate partial region of the lower periodontium, or illuminate simultaneously partial region of the upper periodontium and partial region of the lower periodontium depending on specific needs of the user.

For example, as shown in Fig. 7, the first light-emitting elements 410 and the second light-emitting elements 420 are distributed in a matrix on the base plate 110, wherein three rows and eighteen columns of the first light-emitting elements 410 are provided, and three rows and eighteen columns of the second light-emitting elements 420 are provided. Every three columns of first light-emitting elements 410 may be divided into one first light-emitting matrix, and the first light-emitting elements 410 may be divided into six first light-emitting matrices totally; and every three columns of second light-emitting elements 420 may be divided into one second light-emitting matrix, and the second light-emitting elements 420 may be divided into six second light-emitting matrices in total. The control mechanism may control any one or more first light-emitting matrices to emit light, and the control mechanism may also control any one or more second light-emitting elements 420 to emit light. In this case, according to the user's needs, the illumination may be operated only on the periodontium corresponding to one or several teeth in the oral cavity.

Alternatively, in the process of using, the optical driving mechanism 450 may drive the individual first light-emitting element 410 or the individual second light-emitting element 420 to emit light with a set amplitude, frequency and time, or make the plurality of first light-emitting elements 410 or the plurality of second light-emitting elements 420 simultaneously emit light with a set amplitude, frequency and time, so as to achieve the same effect as the above-mentioned divisional lighting control, which has a wider adjustable range.

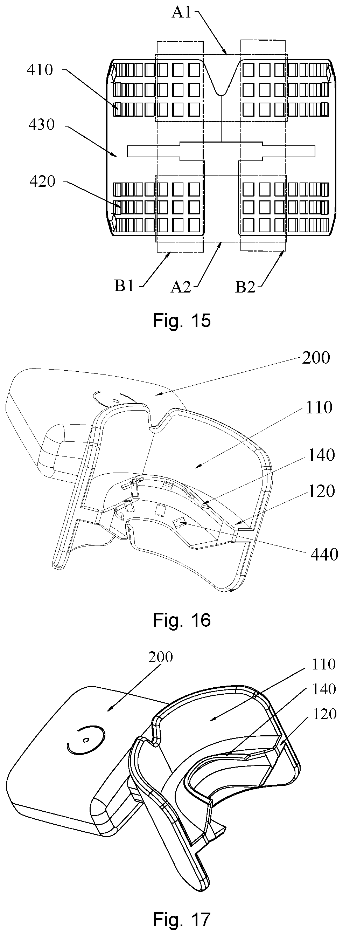

Certainly, the light-emitting matrix may be divided without following the upper region and the lower region, and different light-emitting matrices may contain the same light-emitting element. For example, as shown in Fig. 15, the upper three rows are the first light-emitting elements 410, and the lower three rows are the second light-emitting elements 420. In the process of setting, it may be set that only one of the first light-emitting elements 410 or the second light-emitting elements 420 is turned on within a certain period of time, and it may also light up multiple first light-emitting elements 410 or second light-emitting elements 420 within a certain range, to simultaneously emit light of the same amplitude, frequency and time. For example, a plurality of first light-emitting elements 410 in region A1 shown in Fig. 15 are divided into one light-emitting matrix, and a plurality of second light-emitting elements 420 in region A2 are divided into one light-emitting matrix. During use, the multiple first light-emitting elements 410 in the region A1 synchronously emit light with the same amplitude, frequency and time, and the multiple second light-emitting elements 420 in region A2 emit light with the same amplitude, frequency and time. Alternatively, the plurality of first light-emitting elements 410 in region B1 in Fig. 15 are divided into one light-emitting matrix, and the plurality of second light-emitting elements 420 in region B2 are divided into one light-emitting matrix. During use, the multiple first light-emitting elements 410 in region B1 emit light with the same amplitude, frequency and time synchronously, and the multiple second light-emitting elements 420 in region B2 emit light with the same amplitude, frequency and time.

In the light-emitting matrices of the above-mentioned four regions of A1, A2, B1, and B2, part of the light-emitting elements in the light-emitting matrix of the region A1 belong to the light-emitting matrix of the region B1, and part of the light-emitting elements belong to the light-emitting matrix of the region B2; and part of the light-emitting elements in the light-emitting matrix in the region A2 belong to the light-emitting matrix of the region B1, and part of them belong to the light-emitting matrix of the region B2.

The division for quantity and range of the light-emitting matrix of the light generator 400 mentioned above may be set in various ways before leaving the factory, and the user may choose one of the settings in the process of use. Alternatively, each first light-emitting element 410 and each second light-emitting element 420 may be sequentially numbered according to their positions, and the user may subsume the first light-emitting elements 410 and/or the second light-emitting elements 420 corresponding to certain numbers under a group through the application software on the mobile terminal or the control switch 340 before using.

In a feasible embodiment, the occlusion mechanism 100 is provided with an accommodating cavity, and part of the structure of the vibration conducting sheet 520 is embedded in the accommodating cavity. In this case, the contact area between the vibration conducting sheet 520 and the occlusion mechanism 100 may be larger, and the vibration is transmitted more evenly. Specifically, the accommodating cavity is disposed on the occlusion plate 120.

As shown in Fig. 6, in a preferred embodiment, the vibration conducting sheet 520 includes a first segment 521 and a second segment 522, wherein the first segment 521 and the second segment 522 are of an integral structure, and one end of the first segment 521 is connected to the vibration driving mechanism 510; and the second segment 522 extends into the accommodating cavity, and the shape of plate surface of the second segment 522 is identical or similar to that of the occlusion plate 120. For example, the occlusion plate 120 is an arc plate, and the second segment 522 of the vibration conducting sheet 520 is also of an arc plate structure. On the one hand, this arrangement facilitates inserting the second segment 522 into the occlusion plate 120, and enlarges the contact area between the second segment 522 and the occlusion plate 120 on the other hand.

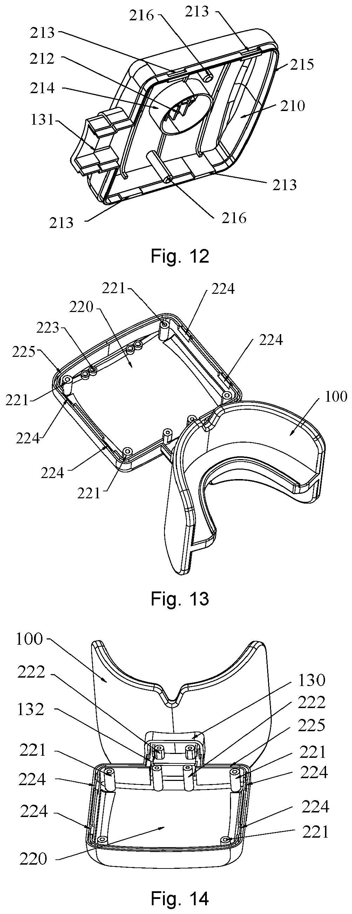

In a feasible embodiment, as shown in Fig. 1 and Fig. 14, the occlusion mechanism 100 is provided with a first insertion portion 130, and the main body housing 200 is provided with a second insertion portion 230. The second insertion portion 230 is inserted into the hole of the first insertion portion 130, wherein the second insertion portion 230 has an inner cavity and the vibration driving mechanism 510 is installed in the inner cavity. In this case, the distance between the vibration driving mechanism 510 and the occlusion mechanism 100 is closer, such that the length of the vibration conducting sheet 520 may be set shorter, thereby reducing energy loss in the process of vibration transmission.

Specifically, the first insertion portion 130 is disposed on a second side surface 112 of the base plate 110, and the first insertion portion 130 is of an annular structure for insertion of the second insertion portion 230. An opening of the accommodating cavity of the occlusion plate 120 is exposed from an inner ring region of the annular structure. On the one hand, the vibration conducting sheet 520 is inserted into the accommodating cavity of the occlusion plate 120 through the interior of the first insertion portion 130, and on the other hand, the connection line between the control mechanism and the light generator 400 passes through the first insertion portion 130 into the base plate 110.

In a specific embodiment, the control mechanism includes a circuit board and a control module. The circuit board is preferably a PCB (printed circuit board) 310, and the control module is integrated on the PCB 310.

As shown in Fig. 6 and Fig. 7, preferably, the light generator 400 is mounted on a flexible circuit board 430. As shown in Fig. 8-Fig. 10, the flexible circuit board 430 is connected to the PCB 310 through a board-to-board connector 330.

In this embodiment, the occlusion mechanism 100 is made of a material with a certain degree of softness, so as to adapt to oral cavity of different shapes and sizes. Preferably, the occlusion mechanism 100 is made of medical silicone or medical rubber. The light generator 400 is embedded inside the base plate 110, and the first side surface 111 of the base plate 110 is made of a transparent material, so as to allow light to emit through the base plate 110.

In a feasible embodiment, the orthodontics assisting device further includes a wireless transmitting module 320, wherein the wireless transmitting module 320 is electrically connected to the control mechanism. The wireless transmitting module 320 is configured to be in signal connection with a mobile terminal, and the mobile terminal is used for inputting control commands to control the control mechanism.

In this case, the control mechanism may be controlled through the mobile terminal, and the using time and using status of the vibration driving mechanism 510 and the optical driving mechanism 450 may be recorded and displayed. For example, the mobile terminal is equipped with application software that cooperates with the orthodontics assisting device. By inputting information in the application software, the working mode may be switched, and the function mode may be customized. For example, illuminating or not may be set in the function mode, and if illumination is performed, any one or more light-emitting elements that emit light or any one or more light-emitting matrices may be further selected. The users may also customize certain light-emitting elements into the same light-emitting matrix. The lighting time, and the wavelength of emitted light may also be selected, and the continuous illumination, the intermittent illumination or alternate illumination between several light-emitting matrices may also be selected. Vibration or not may also be selected, if the vibrating is performed, vibration frequency, vibration amplitude, vibration duration, and continuous vibration or intermittent vibration may be further selected. Optionally, data information such as vibration time, vibration frequency, lighting time, light intensity, treatment time, remaining treatment time, and historical treatment process may be recorded and displayed through the mobile terminal.

The wireless transmitting module 320 may include one or more of a Bluetooth module, a Wi-Fi module, a cellular mobile communication module or other wireless transmitting modules. Preferably, as shown in Fig. 8 to Fig. 10, the wireless transmitting module includes a Bluetooth module. Optionally, when the control mechanism includes PCB 310, the Bluetooth module is integrated into PCB 310.

Optionally, the PCB 310 is connected with a probe 360, and one end of the probe 360 protrudes from the main body housing 200. The probe 360 is configured to connect the circuit board with the charging electrode. The probe 360 is provided inside with a spring and adopts contact-type conductive connection, which has no need for welding and is convenient for manufacturing and assembly.

Referring to Fig. 8-Fig. 10, in a feasible embodiment, the control mechanism is provided with a control switch 340, and the control switch 340 is configured to switch the working modes through the control mechanism, wherein the working modes include at least vibration mode, lighting mode and combined vibration-and-lighting mode.

Optionally, the vibration mode further includes multiple vibration levels, wherein different vibration levels correspond to different vibration frequencies, or different vibration levels correspond to different amplitudes. The lighting mode also includes multiple lighting levels, and different lighting levels correspond to different illumination regions, or different lighting levels correspond to light with different light intensities.

In this way, the start-stop and the mode selection of the orthodontics assisting device may be controlled through the control switch 340, and the control switch 340 may be used by cooperating with a mobile terminal.

The control switch 340 may be push-type, rotary-type, toggle-type or touch-type. In a specific embodiment, as shown in Fig. 8 to Fig. 10, the control switch 340 is a push-type switch. The control switch 340 is integrated on the PCB 310. Correspondingly, as shown in Fig. 1 and Fig. 3, on the main body housing 200, a pressing portion 211 is provided in the region opposite to the control switch 340, and one end of the pressing portion 211 is a free end. By pressing the pressing portion 211 downwards, the pressing portion 211 moves towards the inner side of the main body housing 200 to touch the control switch 340, so as to execute the control operation.

Optionally, as shown in Fig. 11 and Fig. 12, a protruding block 212 is provided on an inner side of the pressing portion 211 (i.e., the side of the pressing portion 211 facing the control switch 340). The setting of the protruding block 212 shortens the distance between the pressing portion 211 and the control switch 340.

As shown in Fig. 1 and Fig. 11, the pressing portion 211 may be formed by cutting an arc-shaped groove on the main body housing 200, and the central angle corresponding to the arc-shaped groove is larger than 270 degrees.

Optionally, as shown in Fig. 8 and Fig. 10, the control mechanism is connected with an indicator light 350.

The indicator light 350 is configured for status indication, wherein the control mechanism may send a control signal to the indicator light 350, such that the indicator light 350 indicates the vibration state, the lighting state, the combined lighting-and-vibration state, the standby state, the undervoltage state, time interval prompt information and other states by changing the color and flickering of light.

The indicator light 350 may be installed on the surface of the first casing or the surface of the second casing; or the indicator light 350 may be installed on the control mechanism, that is, the indicator light 350 is located inside the main body housing 200, and the light of the indicator light 350 may pass through the main body housing 200. Specifically, the main body housing 200 may be made of a transparent material, and the inner side of the main body housing 200 is coated with a light-shielding layer, while the region corresponding to the indicator light 350 is not coated with a light-shielding layer, so that only the indicator light 350 may be seen from outside of the main body housing 200 and other structures may not be seen. Alternatively, a through hole 223 is provided on the main body housing 200, and the light of the indicator light 350 may emit through the through hole 223. Alternatively, an arc-shaped through hole 223 is provided on the main body housing 200, and the region surrounded by the arc-shaped through hole 223 is the pressing portion 211. Part of the structure of the pressing portion 211 is connected with other parts of the main body housing 200, and part of the pressing portion acts as a free end, such that the pressing portion 211 may be pressed inwardly, so as to make the pressing portion 211 press the control switch 340 located inside the main body housing 200. The indicator light 350 may be installed around the control switch 340, so that the light of the indicator light 350 is emitted through the arc-shaped through hole 223.

For example, in a specific embodiment, an indicator light 350 is integrated on the circuit board, and the indicator light 350 may be an LED light bead. The indicator light 350 is located next to the control switch 340. When the control action is performed through the control switch 340, the indicator light 350 may give feedback by changing the on-off state, changing the flickering frequency or changing the color, so that the user knows whether the control action is successful and the working status.

Optionally, a limiting ring 214 is provided at the inner side of the main body housing 200, and the limiting ring 214 is located in the peripheral area of the pressing portion 211. After the main body housing 200 is assembled with the control mechanism, the control switch 340 and the indicator light 350 are both located inside the limiting ring 214, and the limiting ring 214 limits and protects the control switch 340 and the indicator light 350. At the same time, as the limiting ring 214 surrounds the control switch 340 and the indicator light 350, in one aspect, the touch control for the control switch 340 through the pressing portion 211 is not affected; and in another aspect, the light of the indicator light 350 is more gathered, and the light is more obvious.

In a feasible embodiment, as shown in Fig. 11-Fig. 14, the main body housing 200 includes a first half casing 210 and a second half casing 220, and the edge region of the first half casing 210 is snap-fitted to an edge region of the second half casing 220. As shown in Fig. 12, a limiting plug 215 is provided on the edge of the first half casing 210. As shown in Fig. 13 and Fig. 14, a limiting slot 225 is provided on the edge of the second half casing 220, and when the first half casing 210 and the second half casing 220 are combined to each other, the limiting plug 215 extends into the limiting slot 225.

Optionally, a snapping slot 213 is provided on the side of the first half casing 210, and a fixture block 224 is provided on the side of the second half casing 220, and the fixture block 224 may extend into the snapping slot 213.

As shown in Fig. 11 and Fig. 12, both the limiting ring 214 and the pressing portion 211 are disposed in the first half casing 210, and the through hole 223 for the probe 360 to be protruded is disposed on the second half casing 220.

The first half casing 210 includes a first insertion end 131, the second half casing 220 includes a second insertion end 132, wherein the first insertion end 131 and the second insertion end 132 are combined together to form a first insertion portion 130. As shown in Fig. 11, the end surface of the distal end of the first insertion end 131 is an arc surface, which matches the arc surface of the second side surface 112 of the base plate 110. Correspondingly, the end surface of the distal end of the second insertion end 132 is also an arc surface, so that the end surface of the first insertion portion 130 formed by combining the first insertion end 131 and the second insertion end 132 is an arc surface. In this case, after the first insertion portion 130 is inserted into the second insertion portion 230, the contact region between the end surface of the first insertion portion 130 and the base plate 110 is larger, and thus the connection stability is better.

As shown in Fig. 1 and Fig. 6, a pressing portion 211 is provided on the first half casing 210, and a through hole 223 is provided on the second half casing 220, and one end of the probe 360 extends out of the second half casing 220 through the through hole 223.

In a specific embodiment, a first positioning post 221 is provided on the second half casing 220, the first positioning post 221 is provided on the plate surface of the circuit board having a first positioning hole 311, and the first positioning post 221 passes through the first positioning hole 311. A limiting post 216 is provided on the first half casing 210, and a limiting opening 312 is provided on the circuit board, and the limiting post 216 passes through the limiting opening 312. The second half casing 220 limits the position of the circuit board through the first positioning post 221, and the first half casing 210 limits the position of the circuit board through the limiting post 216.

Optionally, as shown in Fig. 8 and Fig. 9, the power supply mechanism 600 is located in the second half casing 220. The control switch 340, the wireless transmitting module 320, the indicator light 350 and the board-to-board connector 330 are all arranged on a side of the PCB 310 away from the power supply mechanism 600. Such arrangement makes the structure more compact, taking up less space and reducing the overall volume of the structure. Optionally, when installing the first half casing 210 and the second half casing 220, the limiting post 216 on the first half casing 210 abuts against the power supply mechanism 600 after passing through the limiting opening 312 on the PCB 310, and the power supply mechanism 600 may be limited to a certain extent by the limiting post 216.

Optionally, a second positioning post 222 is provided on the second half casing 220, a second positioning hole 523 is provided on the vibration conducting sheet 520, a third positioning hole 511 is provided on the vibration driving mechanism 510, and at least one second positioning post 222 passes through the second positioning hole 523 after passing through the third positioning hole 511. The vibration conducting sheet 520 and the vibration driving mechanism 510 are limited in the horizontal direction by the second positioning column 222.

Alternatively, in another specific embodiment, the first positioning post 221 and the second positioning post 222 can be of hollow structure, the opening of the hollow chamber of the first positioning post 221 is opposite to the first positioning hole 311, and the opening of the hollow chamber of the second positioning post 222 is opposite to the second positioning hole 523. Both the hollow first positioning post 221 and the second positioning post 222 are used for screws to pass through, so that the second half casing 220 is connected to the circuit board by screws, and the vibration driving mechanism 510, the vibration conducting sheet 520 and the second half casing 220 are connected by screws.

The above-mentioned orthodontics assisting device (hereinafter referred to as the device) at least has the following working process during use:
clicking the pressing portion 211, such that the pressing portion 211 touches the control switch 340, so as to start the device; and
touching the control switch 340 again by clicking the pressing portion 211 to select the working mode.

After finishing selecting the working mode, the indicator light 350 flashes once every one second, and the device starts working by the selected working mode to work after five seconds. During the working process, every 60 seconds, the vibration driving mechanism 510 drives the occlusion mechanism to vibrate to alert once (if the working mode is the vibration mode, when the vibrating alert is performing, the vibration frequency or vibration amplitude is greater than that of the vibration mode, so that the user can identify this reminder), and the indicator light 350 flashes once, so as to remind user the progress of the working mode. 5 minutes later, the device automatically stops working and enters a standby state, and the indicator light 350 is off.

Another embodiment of the present disclosure provides an orthodontics assisting device, and this embodiment is a further improvement on the basis of the foregoing embodiments. The technical solutions described in the foregoing embodiments also belong to this embodiment, and the technical solutions described in foregoing embodiments will no longer be described repeatedly.

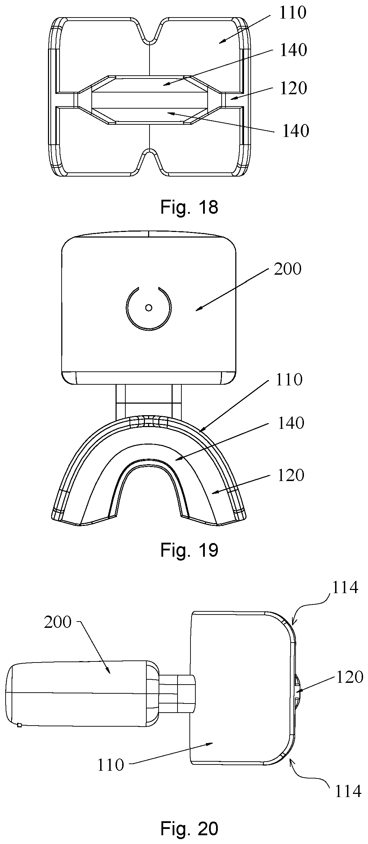

As shown in Fig. 15-Fig. 20, in the orthodontics assisting device provided in this embodiment, an auxiliary lighting board 140 is connected to a side of the occlusion plate 120 facing away from the base plate 110, and the auxiliary lighting board 140 is also provided with one or more light-emitting elements. For the convenience of distinguishing, the light generator 400 arranged on the auxiliary lighting board 140 is called as a third light-emitting element 440, wherein the third light-emitting element 440 is connected with the optical driving mechanism 450, and the third light-emitting element 440 is configured for illuminating the periodontal region facing the inside of the oral cavity.

One or more third light-emitting elements 440 arranged in the auxiliary lighting board 140 are also respectively connected with the optical driving mechanism 450, and the optical driving mechanism 450 controls each third light-emitting element 440, independently; or the third light-emitting elements 440 are subsumed under one or more light-emitting matrices, and each light-emitting matrix is controlled individually. The range of the light-emitting matrix can be determined by the third light-emitting element 440 together with the first light-emitting element 410 and the second light-emitting element 420. That is to say, the third light-emitting element 440 may be located in the same light-emitting matrix with the first light-emitting element 410 and the second light-emitting element 420.

In a feasible embodiment, the base plate 110, the occlusion plate 120 and the auxiliary lighting board 140 are of an integral structure, which can be integrally molded during the manufacturing process. The side of the base plate 110 that is close to the occlusion plate 120 and the side of the auxiliary lighting board 140 that is close to the occlusion plate 120 are made of transparent materials. The light generator 400 is installed on the inside of the base plate 110 and the auxiliary lighting board 140. The light from the light generator 400 passes through the corresponding base plate 110 or the auxiliary lighting board 140 and then exits. The light generator 400 located inside the base plate 110 is configured to illuminate the outer surface of the tooth and the periodontium (such as gingiva) on this surface, and the light generator 400 located in the auxiliary lighting board 140 is configured to illuminate the inner surface of the tooth and the periodontium (such as gingiva) on this surface.

As shown in Fig. 16-Fig. 18, auxiliary lighting boards 140 are provided on both opposite side edges of the occlusion plate 120 in the thickness direction, and the two auxiliary lighting boards 140 are arranged symmetrically with respect to the plane of the plate surface of the occlusion plate 120. One of the auxiliary lighting boards 140 is arranged opposite to the upper region of the base plate 110, wherein the third light-emitting elements 440 in the auxiliary lighting board 140 cooperate with the first light generator 400 in the base plate 110, to illuminate the upper teeth and both the inside and the outside of the periodontium thereof, respectively. Another auxiliary lighting board 140 is opposite to the lower region of the base plate 110, wherein the third light-emitting elements 440 in the auxiliary lighting board 140 cooperate with the second light-emitting elements 420 in the base plate 110, to illuminate the lower teeth and both the inside and the outside of the periodontium thereof, respectively.

In a preferred embodiment, the plate surface of the auxiliary lighting board 140 extends along an arc track both horizontally and vertically, to match the inside of the oral cavity. Thus, the plate surface of the auxiliary lighting board 140 can fit more tightly with the region in the oral cavity located on the inside of the teeth, so that the distance between the third light-emitting elements 440 and the inner side of the teeth and the periodontium on this side is decreased. Optionally, in the longitudinal direction, the middle region of the auxiliary lighting board 140 extends along the arc track for a longer length relative to the regions on either side.

Optionally, there may be multiple third light-emitting elements 440, and each third light-emitting element 440 is mounted on the flexible circuit board 430 and connected to the optical driving mechanism 450 through the flexible circuit board 430. The optical driving mechanism 450 can independently control the amplitude, frequency and time of the light emitted by each third light-emitting element 440. That is to say, the optical driving mechanism 450 can independently control the amplitude, frequency and time of the light emitted by any light generator 400 located in the orthodontics assisting device, regardless that the installation position of the light generator 400 is on the base plate 110 or on the auxiliary lighting board 140.

The above descriptions are only preferred embodiments of the present disclosure, and are not intended to limit the present disclosure. For those of skilled in the art, there may be various modifications and changes in the present disclosure. Any modifications, equivalent replacements, improvements, etc. made within the spirit and principle of the present disclosure shall be included within the protection scope of the present disclosure.

### Industrial Applicability

The present disclosure provides an orthodontics assisting device which can illuminate the periodontium and vibrate the teeth, and user can choose a single vibration mode, a lighting mode or a combined vibration-and-lighting mode according to the needs, so as to increase the moving speed of the teeth, thereby speeding up the orthodontic speed, and reducing tooth pain.

Furthermore, it can be understood that the orthodontics assisting device provided herein can be used in industrial applications and are reproducible.

## Claims

1. An orthodontics assisting device, **characterized in that** the orthodontics assisting device comprises an occlusion mechanism, a control mechanism, a vibration driving mechanism and an optical driving mechanism, the occlusion mechanism is configured to be placed in an oral cavity, and the occlusion mechanism is equipped with a light generator, and the light generator is arranged correspondingly to a position of a periodontium in the oral cavity; the occlusion mechanism is connected to the vibration driving mechanism, and the light generator of the occlusion mechanism is connected to the optical driving mechanism; the vibration driving mechanism and the optical driving mechanism are respectively electrically connected with the control mechanism; the control mechanism is configured to control the vibration driving mechanism to drive the occlusion mechanism to vibrate; and the control mechanism is configured to control the optical driving mechanism to drive the light generator to generate light.

2. The orthodontics assisting device according to claim 1, wherein the light emitted by the light generator has a wavelength of 650 nm to 950 nm.

3. The orthodontics assisting device according to claim 1, wherein the orthodontics assisting device further comprises a main body housing, wherein both the control mechanism and the vibration driving mechanism are installed in the main body housing; and the vibration driving mechanism and the occlusion mechanism is connected through a vibration conducting sheet.

4. The orthodontics assisting device according to claim 3, wherein the main body housing is made of ABS+PC synthetic material.

5. The orthodontics assisting device according to claim 3, wherein the occlusion mechanism is provided with an accommodating cavity, and a part of structure of the vibration conducting sheet is embedded in the accommodating cavity.

6. The orthodontics assisting device according to any one of claims 1 to 5, wherein the orthodontics assisting device further comprises a power supply mechanism, the power supply mechanism is connected with the control mechanism, the vibration driving mechanism and the optical driving mechanism, respectively; and
the power supply mechanism comprises a battery pack, and a voltage stabilizing circuit and a power regulation circuit that are electrically connected to the battery pack.

7. The orthodontics assisting device according to any one of claims 1 to 6, wherein the occlusion mechanism comprises a base plate and an occlusion plate, and a first side surface of the base plate is configured to match a shape of a periodontium in the oral cavity, a corner of each of the base plate and the occlusion plate is provided with a chamfer, the chamfer is a rounded chamfer or a gradual chamfer composed of multiple planes; and the occlusion plate and the base plate are connected at a set angle, and both the light generator and the occlusion plate are mounted on the first side surface of the base plate; and
two opposite edges of the base plate are provided with V-shaped notches recessed from outside to inside, and the two V-shaped notches are respectively located on two sides of the occlusion plate.

8. The orthodontics assisting device according to claim 7, wherein the occlusion plate divides the base plate into an upper region and a lower region, the upper region is configured to correspond with an upper periodontium, and the lower region is configured to correspond with a lower periodontium; and the light generator comprises a plurality of light-emitting elements, and the upper region and the lower region are respectively provided with several light-emitting elements.

9. The orthodontics assisting device according to claim 8, wherein the occlusion plate is further connected with an auxiliary lighting board, the auxiliary lighting board is provided with the light-emitting elements, and the light-emitting elements of the auxiliary lighting board are configured to illuminate a periodontal region toward an inner side of the oral cavity.

10. The orthodontics assisting device according to claim 8 or 9, wherein the plurality of light-emitting elements form several light-emitting matrices, and the plurality of light-emitting elements are respectively connected with the optical driving mechanism; and the optical driving mechanism is capable of independently controlling each of the light-emitting elements, or independently controlling each of the light-emitting matrices composed of the light-emitting elements.

11. The orthodontics assisting device according to any one of claims 3 to 5, wherein the occlusion mechanism is provided with a first insertion portion, and the main body housing is provided with a second insertion portion, wherein the second insertion portion is inserted into a hole of the first insertion portion, the second insertion portion has an inner cavity, and the vibration driving mechanism is installed in the inner cavity.

12. The orthodontics assisting device according to any one of claims 1 to 11, wherein the orthodontics assisting device further comprises a wireless transmitting module, the wireless transmitting module is electrically connected to the control mechanism; and the wireless transmitting module is configured to be in signal connection with a mobile terminal, wherein the mobile terminal is at least configured to input control instructions to control the control mechanism and to record and display relevant data information of a vibration time, a vibration frequency, a lighting time, a light intensity, a treatment time, a remaining treatment time and a historical treatment process of the orthodontics assisting device.

13. The orthodontics assisting device according to claim 12, wherein the wireless transmitting module comprises a Bluetooth module, a Wi-Fi module, a cellular mobile communication module or other wireless transmitting modules.

14. The orthodontics assisting device according to any one of claims 1 to 13, wherein the control mechanism is provided with a control switch, and the control switch is configured to switch working modes through the control mechanism, wherein the working modes at least comprise a vibration mode, a lighting mode and a combined vibration-and-lighting mode; and
the control mechanism is further connected with an indicator light.
